# EUROPÄISCHE PATENTSCHRIFT

(11) **EP 0 591 712 B1**
(45) Veröffentlichungstag und Bekanntmachung des Hinweises auf die Patenterteilung: **17.01.1996**
(21) Anmeldenummer: 93114699.7
(22) Anmeldetag: 13.09.1993
(51) Int. Cl.: A61B 19/00, A61B 6/12

(54) **Vorrichtung zum Markieren von Körperstellen für medizinische Untersuchungen**
Device for marking points on the body used for medical examination
Dispositif de marquage de points de référence sur le corps pour examen médical

(30) Priorität: 08.10.1992 DE 4233978
(43) Veröffentlichungstag der Anmeldung: 13.04.1994
(73) Patentinhaber: Leibinger GmbH, D-79111 Freiburg (DE)
(72) Erfinder: Leibinger, Karl, D-78532 Tuttlingen (DE); Leibinger, Franz, D-78570 Mühlheim-Stetten (DE); Felber, Stephan, Dr., A-6071 Aldrans (AT); Plangger, Clemens, Dr., A-6410 Telfs (AT)
(74) Vertreter: von Hellfeld, Axel, Dr. Dipl.-Phys.

(56) Entgegenhaltungen:
- GB-A- 2 212 371
- US-A- 4 985 019

## Beschreibung

Die Erfindung betrifft eine Vorrichtung zum Markieren von Körperstellen für bilderzeugende medizinische Untersuchungsverfahren, wie insbesondere Kernspintomographie, Computertomographie, Röntgen- oder PET, mit am Körper und/oder einer stereotaktischen Einrichtung befestigbaren Halterungen und jeweils an den Halterungen befestigbaren Markern, die eine bei der Bilderzeugung kontrastreiche Substanz enthalten.

Insbesondere für stereotaktische Operationen, also Gehirnoperationen, bei denen mit Hilfe eines am Kopf des Patienten befestigten Zielgerätes eine Sonde oder Elektrode durch eine kleine in den knöchernen Schädel gebohrte Öffnung unter Schonung benachbarter empfindlicher Strukturen millimetergenau zu einer tiefliegenden Hirnbahn oder einem Nervenkern vorgeschoben wird, sind hochpräzise Untersuchungen über die anatomische Struktur erforderlich.

Hierzu werden sogenannte Markersysteme am Patienten angebracht, wie aus GB-A-2212371 bekannt.

Die Erfindung hat das Ziel, eine Vorrichtung zum Markieren von Körperstellen für bilderzeugende medizinische Untersuchungsverfahren zu schaffen, mit der unterschiedliche Untersuchungsverfahren in einfacher Weise einander ergänzend eingesetzt werden können.

Erfindungsgemäß wird diese Aufgabe dadurch gelöst, daß die Halterungen des Markersystems aus einem Material bestehen, das bei zumindest zwei verschiedenen bilderzeugenden Untersuchungsverfahren die Bild- erzeugung nicht stört.

Solche Markersysteme für die rahmenlose Stereotaxie werden am Patienten angebracht. Sie bestehen aus den oben genannten Halterungen und den eigentlichen Markern, d. h. denjenigen Elementen, die eine Substanz enthalten, welche für das durchzuführende medizinische Untersuchungsverfahren besonders kontrastreich ist, um auf dem Bild deutlich zu erscheinen. Die Halterungen sind dabei so ausgeführt, daß sie implantiert, auf die Körperoberfläche aufgeklebt, an Maskensystemen befestigt oder an Stereotaxiesystemen angebracht werden können. Die Marker (Markierer) selbst lassen sich dann an den Halterungen auswechselbar befestigen. Die Marker enthalten dabei jeweils eine Substanz oder bestehen im wesentlichen aus einer solchen Substanz, die einen guten Bildkontrast in dem bildgebenden Verfahren aufweist. Als bildgebendes Verfahren kommen insbesondere heute in Betracht die Kernspintomographie, die Computertomographie, die Röntgenanalyse oder die PET-Untersuchung.

Bevorzugt sind erfindungsgemäß die Marker auch so ausgeführt, daß sie optisch mit einer Videokamera aufnehmbar und/oder mit einem Operationsmikroskop erkennbar sind. Das Operationsmikroskop ermöglicht eine Positionsrückmeldung, mit der eine genaue Lokalisierung ermöglicht ist.

Mit mindestens drei unterschiedlich positionierten Markern kann die Lage eines Patienten, insbesondere auch die Lage seines Kopfes, im Raum bestimmt werden. Werden nun am Patienten unterschiedliche Untersuchungen mit verschiedenen bilderzeugenden medizinischen Untersuchungsverfahren, wie sie oben beispielhaft aufgeführt sind, durchgeführt, so kann sowohl die Untersuchung als auch gegebenenfalls ein chirurgischer Eingriff dadurch verbessert bzw. erleichtert werden, daß unterschiedliche Untersuchungsverfahren einander ergänzend eingesetzt werden. Ist für die mit verschiedenen Verfahren gewonnenen Bilder jeweils die relative Position des Patienten (z. B. seines Kopfes) genau bestimmt, dann können die verschiedenen bilderzeugenden Untersuchungsverfahren einander überlagert werden, z.B. rein rechnerisch in einem Computer.

Durch die unterschiedlichen Eigenschaften der verschiedenen bilderzeugenden medizinischen Untersuchungsverfahren werden unterschiedliche anatomische Informationen gewonnen. Beispielsweise können mit der digitalen Subtraktionsangiographie gute Röntgenbilder der Blutgefäße im Gehirn hergestellt werden. Die Kernspintomographie liefert häufig gute Bilder von Ödemen. Die Computertomographie hingegen liefert gute Darstellungen der Knochenstrukturen sowie die für eine Strahlentherapie nötigen Dichteinformationen.

Die Erfindung ermöglicht es, unter hohen Genauigkeitsanforderungen, wie sie insbesondere im Kopfbereich gelten, die mit verschiedenen Bilderzeugungsverfahren gewonnenen Ergebnisse einander zu überlagern und/oder zu vergleichen.

Die oben skizzierte erfindungsgemäße Lösung erreicht, daß die erfindungsgemäßen Marker sich ohne Ortsveränderung in verschiedenen bildgebenden Verfahren kontrastreich darstellen. Es werden Halterungen für die Marker verwendet, die sich durch Form und Material so auszeichnen, daß sie die Abbildung der Marker und der anatomischen Strukturen nicht nennenswert stören.

Die Marker sind so ausgelegt, daß sie für verschiedene bildgebende Verfahren jeweils ein geeignetes Material für ihre Hülle sowie das eigentliche Kontrastmittel aufweisen. Solche Marker können dann wahlweise je nach dem bildgebenden Verfahren ausgewechselt werden, wobei die Halterungen unverändert bleiben.

Die Halterungen, die Marker und das Kontrastmittel bestehen aus Materialien, die für den jeweiligen Zweck zugelassen sind. Zu beachten ist dabei, daß nur die Halterungen in direkten Kontakt mit dem Patienten kommen.

Bei sogenannten Projektionsbildern, wie Röntgenaufnahmen, genügen zwei Aufnahmen aus unterschiedlicher Richtung, um für beliebige Punkte eine Ortsbestimmung durchführen zu können.

Bei sogenannten Schnittbildern genügen Aufnahmen, die die Marker sichtbar enthalten, um eine Ortsbestimmung im Raum durchführen zu können.

Für stereotaktisch geführte Eingriffe müssen zur Übertragung der vorbestimmten Zielpunkte auf das Stereotaxiesystem dann noch zusätzliche Aufnahmen gemacht werden, die sowohl die Marker als auch die Position des Stereotaxiesystems sichtbar enthalten. Dabei können unterschiedliche bildgebende Verfahren eingesetzt werden. Auf diese Weise können Zielpunkte und chirurgische Zugangswege festgelegt und auf den Patienten übertragen werden, wobei gleichzeitig ein Stereotaxiesystem in einem der bildgebenden Verfahren oder ein Operationsmikroskop verwendet wird, welches es erlaubt, die Marker anzupeilen und deren Position im Raum zu bestimmen.

Nachfolgend werden mehrere Ausführungsbeispiele von Halterungen und zugeordneten Markern näher beschrieben.

Es zeigt:
- Fig. 1: eine Halterung zum Befestigen von Markern;
- Fig. 2: ein erstes Ausführungsbeispiel eines Markers, der mit einer Halterung gemäß Fig. 1 eingegesetzt werden kann;
- Fig. 3: ein zweites Ausführungsbeispiel eines Markers, der ebenfalls mit einer Halterung gemäß Fig. 1 einsetzbar ist;
- Fig. 4: ein weiteres Ausführungsbeispiel einer Haltetung;
- Fig. 5: noch ein Ausführungsbeispiel einer Halterung;
- Fig. 6: eine Draufsicht auf eine Halterung gemäß Fig. 5;
- Fig. 7: ein anderes Ausführungsbeispiel einer Halterung mit einer Fußplatte;
- Fig. 8: eine Draufsicht auf eine Halterung gemäß Fig. 7;
- Fig. 9: eine Ansicht der Halterung gemäß Fig. 10 in Richtung des Pfeiles P;
- Fig. 10: ein Ausführungsbeispiel einer Halterung, die an einem Stereotaxiesystem befestigbar ist;
- Fig. 11: ein weiteres Ausführungsbeispiel eines Markers, der beispielsweise mit einer der vorstehend genannten Halterungen verwendbar ist;
- Fig. 12: ein Ausführungsbeispiel eines Markers, in den verschiedene Kontrastsubstanzen einbringbar sind;
- Fig. 13: eine Ansicht des Markers gemäß Fig. 12 in Richtung des Pfeiles P;
- Fig. 14: eine Draufsicht auf den Marker gemäß Fig. 12;
- Fig. 15: ein weiteres Ausführungsbeispiel eines Markers mit mehreren Aufnahmen für unterschiedliche Kontrast-Substanzen;
- Fig. 16: einen Axialschnitt durch einen Marker gemäß Fig. 15;
- Fig. 17: einen Schnitt senkrecht zur Längsachse eines Markers gemäß Fig. 15;
- Fig. 18 bis 20: unterschiedliche Ausgestaltungen von Einschüben, die bei Markern gemäß den Fig. 15, 21 und 22 verwendbar sind;
- Fig. 21: ein weiteres Ausführungsbeispiel eines Markers in Abwandlung des Ausführungsbeispiels gemäß Fig. 15 und
- Fig. 22: einen Axialschnitt durch einen Marker gemäß Fig. 21.

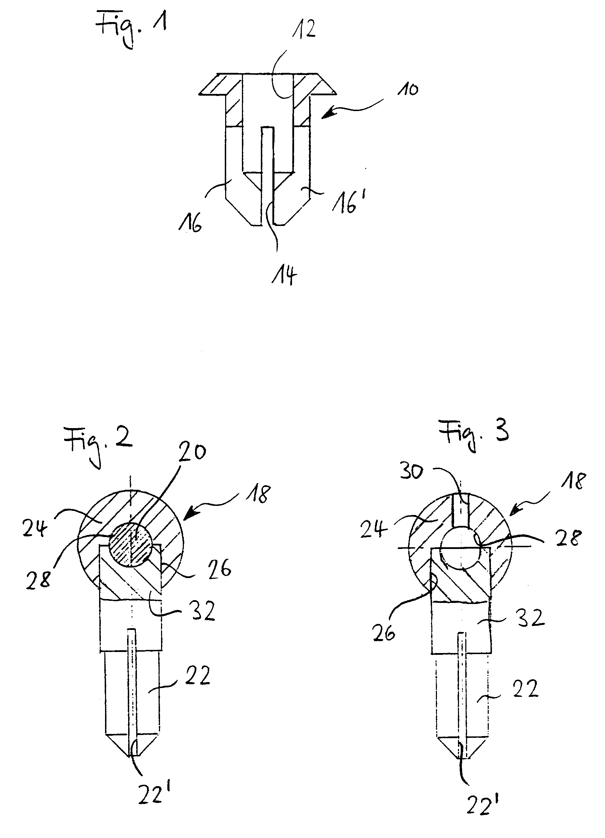

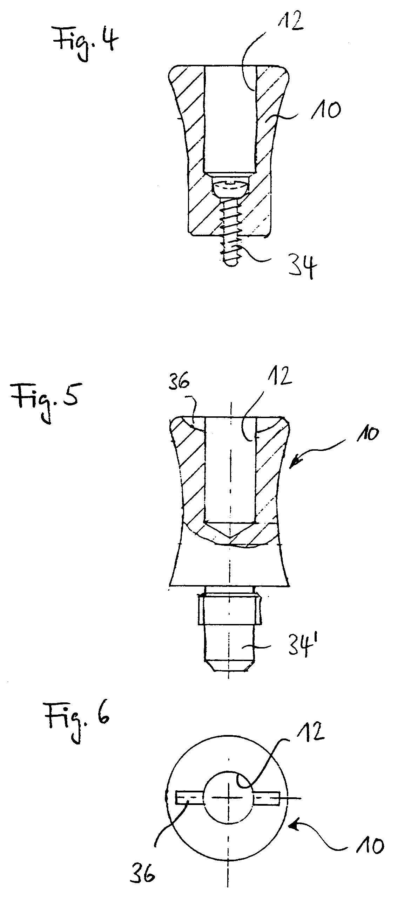

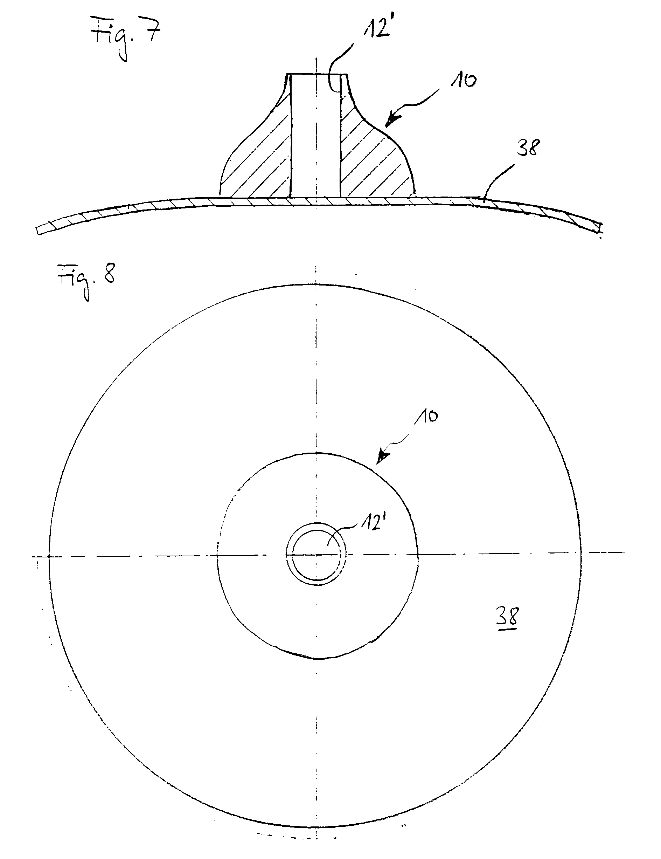

Fig. 1 zeigt eine Halterung, die an einem als solches bekannten Maskensystem befestigbar ist. Die Halterung 10 gemäß Fig. 1 besteht aus Kunststoff und weist eine Ausnehmung 12 auf, in welche ein weiter unten näher beschriebener Marker einschiebbar ist. Die Halterung aus Kunststoff weist zwei Spreizflügel 16, 16' auf, zwischen denen ein Schlitz 14 freigelassen ist.

Die Fig. 2 und 3 zeigen jeweils einen Marker 18, der mit einer Halterung 10 gemäß Fig. 1 verwendbar ist.

Ein Marker 18 enthält eine kontrastreiche Substanz 20 für das gerade angewandte medizinische bilderzeugende Untersuchungsverfahren. Beispiele für die Materialien sind dem Fachmann bekannt und werden weiter unten noch angegeben.

Der Marker 18 weist einen Fuß 22 auf, der in die Ausnehmung 12 der Halterung 10 paßgenau unter Klemmwirkung einschiebbar ist. Hierzu weist der Fuß 22 einen Schlitz 22' auf, um die Klemmwirkung zu erzielen.

Der Fuß 22 des Markers 18 geht in einen Kopf 24 über, in dem ein Hohlraum 28 zur Aufnahme der kontrastreichen Substanz 20 vorgesehen ist. Gemäß den Figuren 2 und 3 ist im Kopf 24 eine Bohrung 26 vorgesehen, in die ein Stift 32 paßgenau einschiebbar ist.

Beispielsweise erlaubt die Substanz 20 im Kopf 24 einmal eine Computertomographie-Aufnahme und, nach Austausch des Markers, zum anderen eine Röntgenaufnahme. Dabei wird die jeweils verwendete Halterung 10 (Fig. 1) nicht geändert. Dies ist nicht erforderlich, weil das Material, aus dem die Halterung 10 besteht, sowohl die Computertomographie als auch die Röntgenaufnahme nicht stört.

Das Ausführungsbeispiel gemäß Fig. 3 unterscheidet sich vom vorstehend beschriebenen Ausführungsbeispiel nach Fig. 2 dadurch, daß im Kopf 24 ein Kanal 30 ausgebildet ist, durch den eine kontrastreiche Substanz in den Hohlraum 28 injizierbar ist. Dies kann z.B. ein Gadolinium-haltiges Kontrastmittel sein. Danach wird der Kanal 30 mit einem geeigneten Mittel verschlossen.

Der Kanal 30 kann auch so ausgeführt sein, daß die Oberflächenspannung des Kontrastmittels (welches eine Flüssigkeit ist) ein Auslaufen verhindert.

Die Marker 18 gemäß den Fig. 2 und 3 sind hinsichtlich ihrer äußeren Abmessungen gleich und an die Abmessungen der Halterung 10 angepaßt, so daß sie wahlweise und mit jeweils anderen kontrastreichen Substanzen in die Halterung 10 einschiebbar sind.

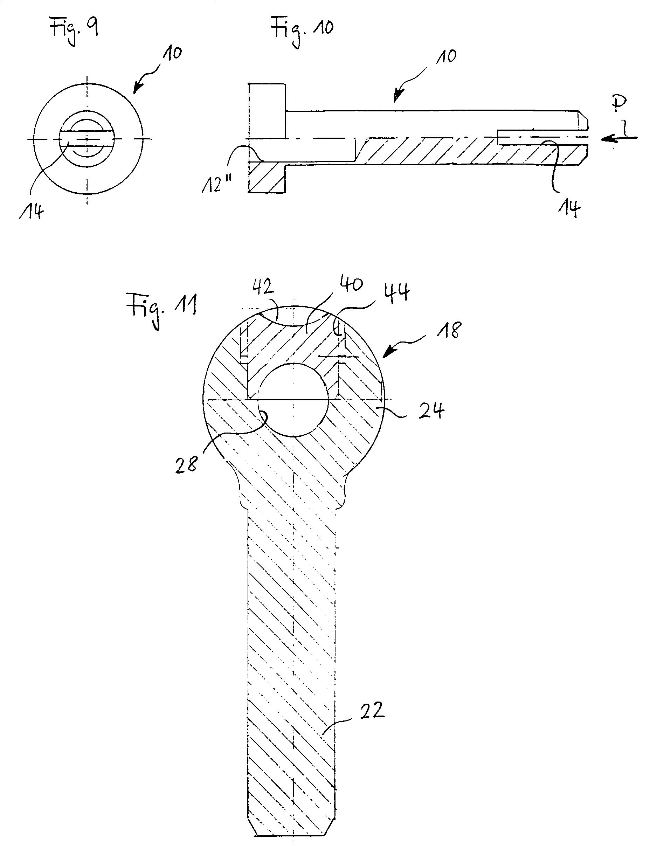

Fig. 4 zeigt eine in den Körper implantierbare Halterung 10. Dabei dient die Ausnehmung 12 wieder zur Aufnahme eines Markers (z. B. entsprechend den Fig. 2 und 3), während eine Schraube 34 dazu dient, die Halterung 10 an einem Knochen, beispielsweise einem Schädelknochen, zu befestigen. Wie bei den obigen Ausführungsbeispielen, besteht auch hier die Halterung 10 aus einem körperverträglichen Kunststoff, während die Schraube 34 eine metallische Knochenschraube ist.

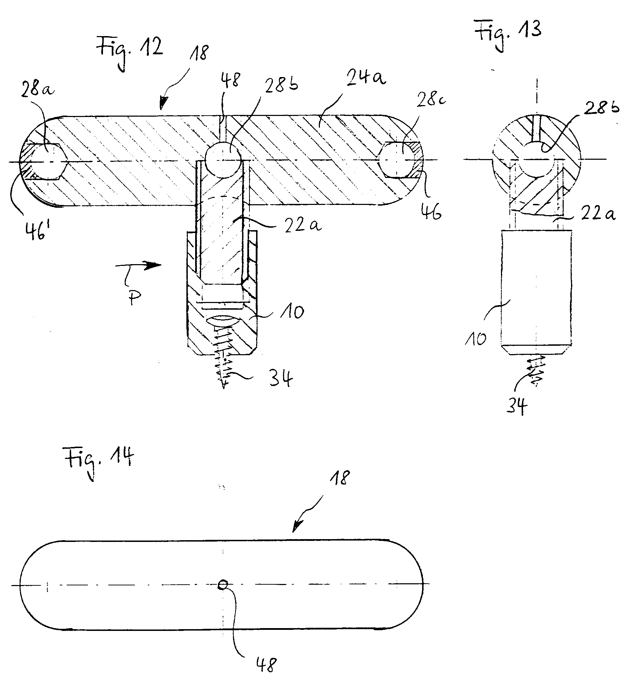

Die Fig. 5 und 6 zeigen eine weitere Ausführungsform eines Halters 10, wobei die Halterung 10 mit einer bereits implantierten Knochenschraube verwendbar ist. Die Halterung 10 wird mit einem Fuß 34' auf die bereits festsitzende Knochenschraube aufgedreht. Am proximalen Ende der Halterung 10 ist ein Schlitz 36 zur Aufnahme eines Schraubendrehers vorgesehen.

Die Fig. 7 und 8 zeigen ein weiteres Ausführungsbeispiel einer Halterung, welche diesmal auf eine Körperoberfläche aufklebbar ist. Die Halterung 10 weist einen etwa glockenförmigen Körper mit einer Ausnehmung 12' auf, in die analog den obigen Ausführungsbeispielen gemäß den Fig. 1, 4 und 5 ein Fuß 22 entsprechend den Fig. 2 und 3 einschiebbar ist. Eine Platte 38 dient der Fixierung der Halterung 10 auf beispielsweise der Haut. Dabei wird die Platte 38 mit einem geeigneten Kleber befestigt.

Fig. 8 zeigt eine Draufsicht des vorstehend beschriebenen Ausführungsbeispiels nach Fig. 7.

Die Fig. 9 und 10 zeigen eine Halterung 10, die an einem Stereotaxiesystem befestigbar ist. Hierzu ist die Halterung 10 in der Form eines Stiftes (Fig. 10) ausgebildet. Dieser Stift kann bis zum Anschlag des dickeren Querschnitts in eine Bohrung des Stereotaxiesystems gesteckt werden. Anschließend kann dann ein Marker 18, z. B. gemäß den Fig. 2 und 3, in die Ausnehmung 12'' eingesetzt werden. Fig. 9 zeigt eine Ansicht auf den Stift in Richtung des Pfeiles P gemäß Fig. 10. Der Stift bildet die Halterung 10 gemäß Fig. 10. Zu erkennen ist ein Schlitz 14, der in einer Bohrung eines Stereotaxiesystems eine Klemmwirkung erzeugt.

Fig. 11 zeigt ein weiteres Ausführungsbeispiel eines Markers 18, also eine Abwandlung der in den Fig. 2 und 3 beschriebenen Marker. Der Fuß 22 dient auch hier wieder der Befestigung des Markers in einer Halterung, beispielsweise entsprechend den Fig. 1 und 4 bis 10. Der Kopf 24 des Markers 18 nach Fig. 11 ist mit einem Deckel 40 versehen, der in abgenommenem Zustand einen Zugang zum Hohlraum 28 freigibt, in den das Kontrastmittel eingebbar ist. Der als Schraubverschluß ausgeführte Deckel 40 ist an seinem Boden mit einer halbkugelförmigen Ausnehmung versehen, welche eine komplementäre halbkugelförmige Ausnehmung im Kopf 24 zu einer Vollkugel ergänzt. Fig. 11 zeigt noch ein Gewinde 44 zum Einschrauben des Deckels 40 in den Kopf 24 und einen Schlitz 42, der zur Aufnahme eines Schraubendrehers dient.

Der Kopf 24 sowie der Fuß 22 und auch der Deckel 40 können aus Teflon bestehen.

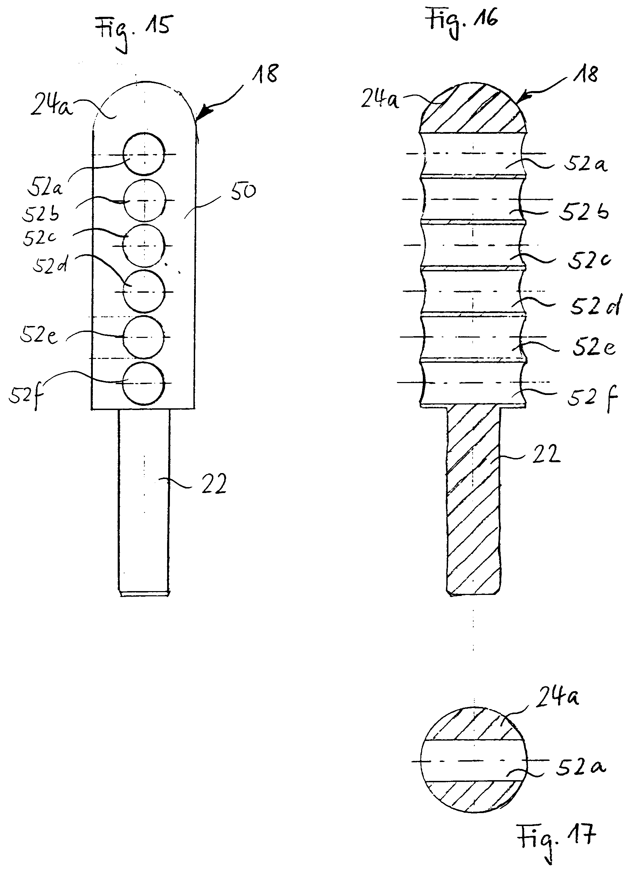

Die Fig. 12, 13 und 14 zeigen ein weiteres Ausführungsbeispiel eines Markers 18. Auch hier wird wieder der Fuß 22a in eine Halterung 10 (z. B. gemäß den Fig. 1, 4 - 10) eingesetzt. Dies ist in den Fig. 12 und 13 dargestellt. Der Marker 18 weist einen sich quer zum Fuß 22a erstreckenden armartigen Querkörper 24a auf, der mehr als einen Hohlraum zur Aufnahme von kontrastreichen Substanzen aufweist, nämlich die Hohlräume 28a, 28b und 28c. Diese Hohlräume können gleichzeitig unterschiedliche Kontrastmittel aufnehmen. Hierzu verbindet ein Kanal 48 den Hohlraum 28b mit der Außenseite. Die Hohlräume 28a, 28c sind jeweils durch Stöpsel 46 bzw. 46' nach außen abdichtbar. Die Stöpsel können beispielsweise verklebt sein. Der geometrische Mittelpunkt der beiden äußeren Hohlräume 28a, 28c fällt mit dem Mittelpunkt des mittleren Hohlraumes 28b zusammen.

Die Fig. 15 bis 17 zeigen ein weiteres Ausführungsbeispiel eines Markers 18, der gleichzeitig verschiedene auswechselbare Kontrastmittel aufnehmen kann. Im Kopf 24a des Markers 18 sind jeweils eine Mehrzahl von durchgehenden, zylinderförmigen Hohlräumen 52a bis 52d ausgebildet, in welche Einschübe entsprechend den Fig. 18 bis 20 einsetzbar sind, in denen jeweils das Kontrastmittel enthalten sein kann. In jeden der Hohlräume 52a bis 52d können also Einschübe 60 entsprechend den Fig. 18 bis 20 eingesetzt werden. Damit können unterschiedliche Kontrastmittel mit jeweils wählbarem Abstand verwendet werden. Der Mittelpunkt der einzelnen einsetzbaren Einschübe mit den markierenden Kontrastmitteln liegt jeweils auf der gleichen Achse, beim dargestellten Ausführungsbeispiel der Längsachse des Markers 18 und seines Fußes 22. Insbesondere läßt sich somit ebenfalls erreichen, daß der geometrische Mittelpunkt zweier äußerer Marker mit dem Mittelpunkt eines mittig angeordneten Markers zusammenfällt.

Die Fig. 18, 19 und 20 zeigen Einschübe 60 zur Verwendung beispielsweise mit einem Marker 18 entsprechend den Fig. 15 bis 17.

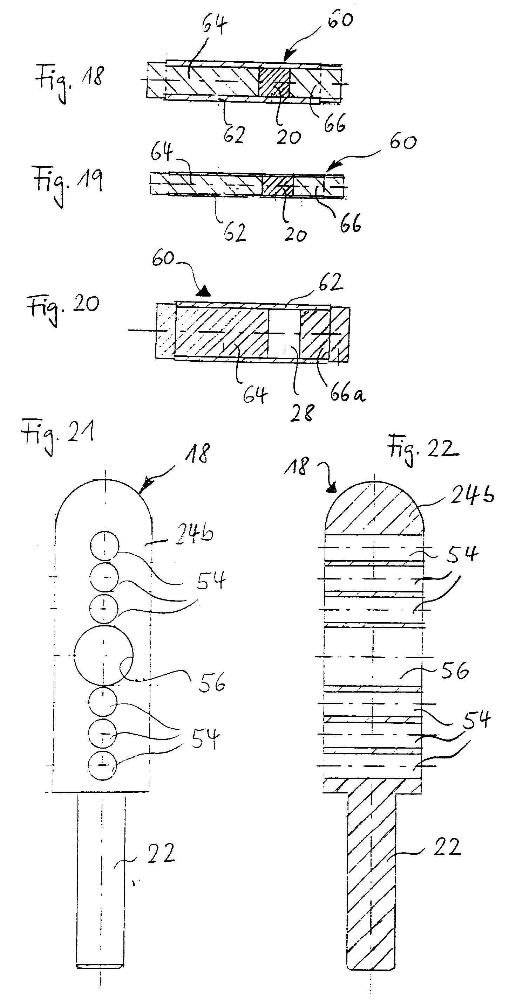

Ein Einschub 60 weist einen äußeren Schlauch 62 auf, beispielsweise aus PTFE. In den Schlauch 62 sind von beiden Enden her jeweils Stangen 64 bzw. 66 so einschiebbar, daß mittig im Schlauch ein Hohlraum verbleibt, in den eine kontrastreiche Substanz (kontrastreich für das gerade verwendete bilderzeugende Untersuchungsverfahren) eingebbar ist. Beim Ausführungsbeispiel gemäß Fig. 20 ist der Hohlraum 28 zunächst ohne Füllung. Die Stangen 64 und 66a sind so ausgebildet, daß sie vom Schlauch 62 abziehbar sind, so daß der Benutzer den Hohlraum 28 wahlweise mit dem gewünschten Kontrastmittel füllen kann.

Die Fig. 21 und 22 zeigen ein weiteres Ausführungsbeispiel eines Markers 18, welches dem Ausführungsbeispiel gemäß den Fig. 15 und 16 ähnlich ist, wobei jedoch Einschübe unterschiedlicher Größe in die einzelnen Hohlräume einsetzbar sind. In die Hohlräume 54 sind Einschübe mit geringerem Durchmesser einsetzbar, während in den Hohlraum 56 ein Einschub größeren Durchmessers einsetzbar ist. Auch hiermit läßt sich erreichen, daß der geometrische Mittelpunkt zweier äußerer Marker mit dem Mittelpunkt des mittig angeordneten Markers zusammenfällt.

Bei den vorstehend beschriebenen Ausführungsbeispielen sind einander gleiche oder zumindest funktionsähnliche Bauteile jeweils mit den gleichen Bezugsziffern versehen, wobei Abwandlungen mit einem Strich bzw. mit kleinen Buchstaben angedeutet sind.

Als Materialien für die Halterungen, insbesondere die Füße und den Körper der Halterungen entsprechend den Fig. 1, 4, 5, 7 etc. kommt insbesondere Polysulfon in Betracht. Polysulfon ist als Implantat geeignet, sterilisierbar und durchsichtig, so daß es eine optische Zuordnung des Füllmaterials ermöglicht.

Als Werkstoff für einen Marker 18, beispielsweise entsprechend den Fig. 2 und 3, kommt insbesondere Polysulfon oder Metall, wie z. B. Titan in Betracht.

Für den Halter entsprechend Fig. 7 kommt ebenfalls Polysulfon in Betracht und für die Fußplatte 38 ein handelsüblicher Elektrodenklebering.

Eine Halterung entsprechend den Fig. 9 und 10 kann beispielsweise aus Polyamid, Polyamidimid oder einem ähnlichen Kunststoff bestehen.

Ein Marker 18 entsprechend Fig. 11 kann beispielsweise aus PTFE (Teflon) oder PSU oder einem anderen als Implantat geeigneten Kunststoff bestehen.

Aus der obigen Beschreibung von Ausführungsbeispielen ergibt sich, daß die Marker (insbesondere die Marker 18 gemäß den Fig. 2 und 3) sich durch eine Kugelsymmetrie auszeichnen und so in jeder Blickrichtigung gleich abbilden. Dies ermöglicht eine eindeutige Positionsbestimmung in Schnittbildern, Projektionsbildern und auch bei optischen Verfahren.

Die Marker können aufgrund einer Drei- oder Mehrfachsymmetrie gleichzeitig in mehreren bildgebenden Verfahren abgebildet werden, wobei gleichzeitig mehrere Kontrastmittel verwendet werden können (Ausführungsbeispiele gemäß den Fig. 12 und 15 bis 22). Dabei können Marker verwendet werden, die gleichzeitig unterschiedliche Kontrastmittel enthalten.

Insbesondere kann auf diese Weise ein Kontrastmittel verwendet werden, das sich sowohl in der Kernspintomographie als auch in der Computertomographie gut abbildet.

Die dargestellten Ausführungsbeispiele haben auch die Eigenschaft (Fig. 2, 3), daß der Mittelpunkt eines kugelförmigen Kontrastmittels zusammenfällt mit einem eindeutig definierten, optisch erkennbaren Mittelpunkt des Markers 18, der dabei insbesondere kugelförmig ausgestaltet ist.

## Patentansprüche

1. Vorrichtung zum Markieren von Körperstellen für bilderzeugende medizinische Untersuchungsverfahren mit Halterungen (10), die am Körper oder einer stereotaktischen Einrichtung befestigbar sind, und mit Markern (18), die eine bei der Bilderzeugung kontrastreiche Substanz enthalten,
dadurch **gekennzeichnet,** daß
- die Marker (18) abnehmbar an den Halterungen befestigbar sind und
- die Halterungen (10) aus einem Material bestehen, das bei zumindest zwei verschiedenen bilderzeugenden Untersuchungsverfahren die Bilderzeugung nicht stört.

2. Vorrichtung nach Anspruch 1,
dadurch **gekennzeichnet,** daß die Marker (18) auch optisch mit einer Videokamera aufnehmbar und/oder mit einem Operationsmikroskop erkennbar sind.

3. Vorrichtung nach einem der Ansprüche 1 oder 2,
dadurch **gekennzeichnet,** daß wahlweise unterschiedliche, jeweils für verschiedene bilderzeugende Untersuchungsverfahren kontrastreiche Marker (18) an denselben Halterungen (10) befestigbar sind.

4. Vorrichtung nach einem der vorhergehenden Ansprüche,
dadurch **gekennzeichnet,** daß an einer Halterung (10) jeweils in vorgegebener Stellung zumindest zwei verschiedene Marker positionierbar sind, die für jeweils verschiedene bilderzeugende Untersuchungsverfahren kontrastreich sind.

5. Vorrichtung nach einem der vorhergehenden Ansprüche,
dadurch **gekennzeichnet,** daß die Marker (18) keine vorstehenden Spitzen und/oder Vorsprünge aufweisen.

## Claims

1. Device for marking points on the body used for image-generating medical examination procedures, comprising fixtures (10) which can be fastened to the body or a stereo-tactical means and markers (18) which include a high-contrast substance for the image generation,
**characterized in** that
- the markers (18) can detachably be mounted to the fixtures, and
- the fixtures (10) consist of a material which does not disturb the image generation in case of at least two different image-generating examination procedures.

2. Device according to claim 1,
**characterized in** that the markers (18) can also be recorded optically by means of a video camera and/or an operating microscope.

3. Device according to any of claims 1 or 2,
**characterized in** that different high-contrast markers (18), each used for different image-generating examination procedures, may optionally be mounted to the same fixtures (10).

4. Device according to any of the preceding claims,
**characterized in** that at least two different markers can be positioned in given positions at one fixture (10), respectively, said markers being of high contrast for different image-generating examination procedures, respectively.

5. Device according to any of the preceding claims,
**characterized in** that the markers (18) do not comprise any projecting tips and/or protrusions.

## Revendications

1. Dispositif de marquage d'emplacements du corps pour des procédés d'examens médicaux par imagerie à l'aide d'attaches (10) fixables sur le corps ou à un dispositif stéréotaxique et de marqueurs 18 contenant une substance contrastante lors de la production de l'image,
caractérisé en ce que :
- les marqueurs (18) sont fixables de façon amovible aux attaches et
- les attaches (10) sont en un matériau qui ne gêne pas la production de l'image pour au moins deux procédés d'examen par imagerie différents.

2. Dispositif selon la revendication 1, caractérisé en ce que les marqueurs (18) sont également enregistrables optiquement à l'aide d'une caméra vidéo et/ou reconnaissables à l'aide d'un microscope chirurgical.

3. Dispositif selon une des revendications 1 et 2, caractérisé en ce que des marqueurs facultativement différents, contrastants pour divers procédés d'examens par imagerie, sont fixables aux mêmes attaches (10).

4. Dispositif selon une des revendications précédentes, caractérisé en ce qu'au moins deux marqueurs différents contrastants pour divers procédés d'examens par imagerie, sont positionnables dans une position prédéterminée sur une attache (10).

5. Dispositif selon une des revendications précédentes, caractérisé en ce que les marqueurs (18) ne présentent aucune pointe et/ou saillie dépassant.
